# EUROPEAN PATENT SPECIFICATION

(11) **EP 2 445 448 B1**
(45) Date of publication and mention of the grant of the patent: **03.04.2013**
(21) Application number: 10729029.8
(22) Date of filing: 25.06.2010
(51) Int. Cl.: A61F 2/16, A61B 5/04

(54) **ELECTRICAL AMPLIFICATION OF PHYSIOLOGIC SIGNALS FOR ACCOMMODATIVE IOL CONTROL**
ELEKTRISCHE VERSTÄRKUNG PHYSIOLOGISCHER SIGNALE FÜR DIE KONTROLLE EINER AKKOMODATIVEN IOL
AMPLIFICATION ÉLECTRIQUE DE SIGNAUX PHYSIOLOGIQUES POUR LE CONTRÔLE D'UNE LIO ACCOMMODATIVE

(30) Priority: 26.06.2009 US 492218
(43) Date of publication of application: 02.05.2012
(73) Proprietor: Alcon Research, Ltd., Fort Worth, Texas 76134 (US)
(72) Inventor: SCHAPER, JR., Dale Thomas, Lakeside, Texas 76108 (US)
(74) Representative: Hanna, Peter William Derek
(86) International application number: PCT/US2010/039978
(87) International publication number: WO 2010/151757

(56) References cited:
- WO-A2-2006/050171
- WO-A2-2010/003058
- DE-A1-102005 038 542
- US-A- 4 373 218
- US-A- 5 443 506

## Description

### BACKGROUND OF THE INVENTION

The present invention relates to a system for controlling an accommodative intraocular lens and more specifically to a system that senses and amplifies the electrical signals that result from the control or movement of the ciliary muscles.

The human eye functions to provide vision by transmitting light through a clear outer portion called the cornea, and focusing the image by way of a crystalline lens onto a retina. The quality of the focused image depends on many factors including the size and shape of the eye, and the transparency of the cornea and the lens. When age or disease causes the lens to become less transparent, vision deteriorates because of the diminished light which can be transmitted to the retina. This deficiency in the lens of the eye is medically known as a cataract. An accepted treatment for this condition is surgical removal of the lens and replacement of the lens function by an artificial intraocular lens (IOL).

In the United States, the majority of cataractous lenses are removed by a surgical technique called phacoemulsification. A typical surgical hand piece suitable for phacoemulsification procedures consists of an ultrasonically driven phacoemulsification hand piece, an attached hollow cutting needle surrounded by an irrigating sleeve, and an electronic control console. The hand piece assembly is attached to the control console by an electric cable and flexible tubing. Through the electric cable, the console varies the power level transmitted by the hand piece to the attached cutting needle. The flexible tubing supplies irrigation fluid to the surgical site and draws aspiration fluid from the eye through the hand piece assembly.

The operative part in a typical hand piece is a centrally located, hollow resonating bar or horn directly attached to a set of piezoelectric crystals. The crystals supply the required ultrasonic vibration needed to drive both the horn and the attached cutting needle during phacoemulsification, and are controlled by the console. The crystal/horn assembly is suspended within the hollow body or shell of the hand piece by flexible mountings. The hand piece body terminates in a reduced diameter portion or nosecone at the body's distal end. Typically, the nosecone is externally threaded to accept the hollow irrigation sleeve, which surrounds most of the length of the cutting needle. Likewise, the horn bore is internally threaded at its distal end to receive the external threads of the cutting tip. The irrigation sleeve also has an internally threaded bore that is screwed onto the external threads of the nosecone. The cutting needle is adjusted so that its tip projects only a predetermined amount past the open end of the irrigating sleeve.

During the phacoemulsification procedure, the tip of the cutting needle and the end of the irrigation sleeve are inserted into the anterior capsule of the eye through a small incision in the outer tissue of the eye. The surgeon brings the tip of the cutting needle into contact with the lens of the eye, so that the vibrating tip fragments the lens. The resulting fragments are aspirated out of the eye through the interior bore of the cutting needle, along with irrigation solution provided to the eye during the procedure, and into a waste reservoir. An IOL is then implanted in the eye to take the place of the natural lens.

While not yet commercially feasible, substantial work is being done on accommodative IOLs. An accommodative IOL simulates the focusing ability of the natural lens. Just like the natural lens, an accommodative IOL can be adjusted to focus on objects very close to the face or on objects at a great distance. In order to perform this focusing function, an accommodative IOL must be moved or altered in some way.

In the human eye, the ciliary muscles are largely responsible for focusing the natural lens. The ciliary muscles are attached to the lens capsule - a thin elastic membrane enveloping the natural lens. Basically, the ciliary muscles control the tension of the capsule thereby focusing the natural lens. In order to mimic the focusing ability of the natural lens, it would be desirable to harness the signals that control the ciliary muscles, or an electrical signal resulting from the mechanical movement of the ciliary muscles, and use that signal to control an accommodative IOL.

US-5,443,506 A and DE-10 2005 0385542 A1 are representative of the state of the art.

### SUMMARY OF THE INVENTION

In one embodiment consistent with the principles of the present invention, the present invention is a control system for an accommodative intraocular lens in accordance with claims which follow. The control system includes a sensing circuit for sensing a signal that controls a ciliary muscle of an eye and an amplifier circuit for amplifying the sensed signal. The output of the amplifier circuit is used to control an accommodative IOL. At least one electrode located in the vicinity of cranial nerve III, the ciliary ganglion, or the ciliary muscles is used to receive the signal that controls the ciliary muscles.

It is to be understood that both the foregoing general description and the following detailed description are exemplary and explanatory only and are intended to provide further explanation of the invention as claimed. The following description, as well as the practice of the invention, set forth and suggest additional advantages and purposes of the invention.

### BRIEF DESCRIPTION OF THE DRAWINGS

The accompanying drawings, which are incorporated in and constitute a part of this specification, illustrate several embodiments of the invention and together with the description, serve to explain the principles of the invention.
Figure 1 is a block diagram of a system for sensing and amplifying ciliary muscle signals, or a system for sensing ciliary muscle movement for controlling an accommodative IOL according to the principles of the present invention.
Figure 2 is a circuit diagram of a system for sensing, filtering, and amplifying ciliary muscle signals for controlling an accommodative IOL according to the principles of the present invention.
Figure 3 is a flow chart of a method of sensing and amplifying ciliary muscle signals for controlling an accommodative IOL according to the principles of the present invention.

### DETAILED DESCRIPTION OF THE PREFERRED EMBODIMENTS

Reference is now made in detail to the exemplary embodiments of the invention, examples of which are illustrated in the accompanying drawings. Wherever possible, the same reference numbers are used throughout the drawings to refer to the same or like parts.

The inventor has discovered that the electrical signals that control the movement of the ciliary muscles can also be used to control an accommodative IOL. As noted above, the ciliary muscles control the tension on the capsule surrounding the natural lens. Since the natural lens is resilient, the tension of the capsule changes the shape of the natural lens thereby enabling a person to focus his eyes. Cranial nerve III (the oculomotor nerve) carries the parasympathetic signal to the ciliary muscles (synapsing on the ciliary ganglion). This parasympathetic signal controls the movement of the ciliary muscles. The ciliary muscles contract when there is parasympathetic activation of the M3 muscarinic receptors on the ciliary muscles. When the ciliary muscles contract, the tension on the capsule is lessened allowing the natural lens to take on a more spherical shape to accommodate for close vision. When the ciliary muscles relax, the tension on the capsule increases thereby squeezing the natural lens into a flatter shape to provide distance vision.

An implantable circuit that senses and amplifies the signals that control the ciliary muscles, or the movement of the ciliary muscles can be used to control an accommodative IOL. In this manner, the parasympathetic signal that controls the ciliary muscle is sensed by one or more electrodes in the vicinity of cranial nerve III, the ciliary ganglion, or the ciliary muscles. Once sensed, this signal can be amplified and used to control an accommodative IOL. In this manner, the brain's own image processing is used to control an accommodative IOL. Such a control system can be used with any of a number of different electrically-controlled IOLs that are currently being developed.

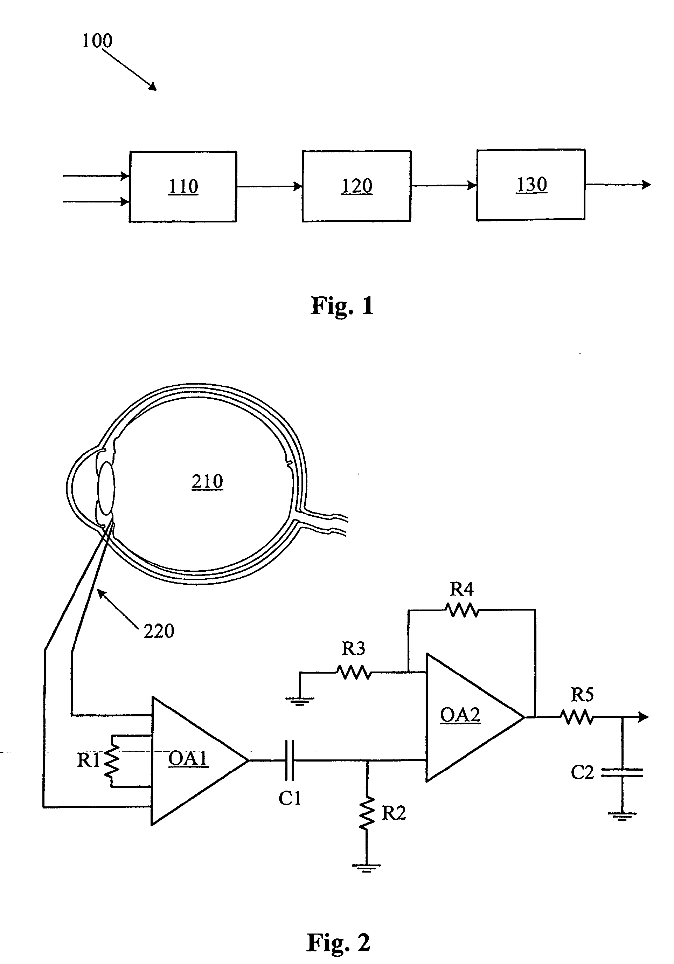

Figure 1 is a block diagram of a system for sensing and amplifying ciliary muscle signals for controlling an accommodative IOL according to the principles of the present invention. In Figure 1, system 100 includes a sensing circuit 110, an optional filter 120, and an amplifier circuit 130. At least one electrode in the vicinity of cranial nerve III, the ciliary ganglion, or the ciliary muscles provides an input to sensing circuit 110. Sensing circuit 110 is electrically coupled to optional filter 120 which is electrically coupled to amplifier circuit 130. The output of amplifier circuit 130 is used to control an accommodative IOL.

Sensing circuit 110 can be implemented in any of a number of different ways. For example, sensing circuit 110 may comprise an op amp whose inputs are connected to one or more electrodes (as shown in Figure 2). Sensing circuit 110 acts to detect the parasympathetic signal via electrodes placed in the vicinity of cranial nerve III, the ciliary ganglion, or the ciliary muscles. The parasympathetic signal carried by cranial nerve III, like other nerve signals, is a very low power signal. Accordingly, sensing circuit 110 is capable of detecting such low power signals. Circuits used to sense other physiologic signals, such as those used in pacemakers, electrocardiograms, or cardioverter defibrillators, are suitable for an implementation of sensing circuit 110.

In another embodiment of the present invention, electrodes may be electrically coupled to an implantable variable resistor that converts movement of the ciliary muscle into an electrical signal. In this manner, the electrical signal from the variable resistor (or other similar device) is used as a control input. The same sensing circuit 110 can be employed to read the signal from the variable resistor (or other similar device).

Like sensing circuit 110, filter 120 can be implemented in any of a number of different ways. Simple RC circuits like those shown in Figure 2 may be employed, as may other passive filter circuits. Active filter circuits are also suitable. The configuration of filter 120 depends on the noise level encountered and the placement of the electrodes. While shown in Figure 1 as being located between sensing circuit 110 and amplifier circuit 130, filter 120 may be placed at any other suitable location. Further, more than one circuit may be used to implement filter 120. In other embodiments, filter 120 is absent.

Like sensing circuit 110 and filter 120, amplifier circuit 130 can be implemented in any of number of different ways. For example, a simple electronic amplifier circuit or operational amplifier (op amp) may be employed. In other embodiments of the present invention, more complicated amplifier circuits can be used. When an op amp is used as amplifier circuit, a very small IC package can be selected.

While shown as separate blocks in Figure 1, sensing circuit 110, filter 120, and amplifier circuit 130 may be combined on a single substrate or in a single IC package.

Figure 2 is a circuit diagram of an exemplary system for sensing, filtering, and amplifying ciliary muscle signals for controlling an accommodative IOL according to the principles of the present invention. In Figure 2, a sensing circuit is implemented with electrodes 220, resistor R1, and op amp OA1. A high pass filter is implemented with capacitor Cl and resistor R2. An amplifier circuit is implemented with op amp OA2, resistor R3, and resistor R4. A low pass filter is implemented with resistor R5 and capacitor C2.

Electrodes 220 are placed in the eye 210 in the vicinity of cranial nerve III, the ciliary ganglion, or the ciliary muscles. Alternatively, electrodes 220 are coupled to an implanted variable resistor (or other similar device) that converts movement of the ciliary muscle to an electrical signal. Typically, electrodes 220 are made of small gauge wire of a suitable length to be properly placed in the eye to read signals that control the ciliary muscle. The length of electrodes 220 is thus dependent on the configuration and placement of the control system in the eye. The distal end of electrodes 220 may be sharp to facilitate placement in the eye and may be barbed to ensure that they are not dislodged.

Op amp OA1 and resistor R1 act as a sensing circuit. The input of OA1 is electrically coupled to the electrodes 220. The output of OA1 is a representation of the signal carried by cranial nerve III that controls the ciliary muscles. This signal is then filtered by a high pass filter (C1 and R2) before entering the input of OA2. OA2, R3, and R4 act to amplify the filtered signal. As is commonly known, the gain of the amplifier is dependent on the selection of R3 and R4 (gain = 1 + R4/R3). The output of OA2 is thus an amplified and filtered signal that represents the signal carried by cranial nerve III that controls the ciliary muscles. This amplified signal is then filtered by a low pass filter (R5 and C2) before being used to control an accommodative IOL.

The circuit of Figure 2 can be modified in numerous different ways. For example, a band pass filter may be used in place of the high pass and low pass filters. The band pass filter could be located between the sensing circuit and the amplifier circuit (between the output of OA1 and the input of OA2), or it could be located at the output of the amplifier circuit (at the output of OA2). Other similar modifications can also be made to the implementation shown in Figure 2.

Regardless of the types of circuits used to implement system 100, the overall size and shape of system 100 is suitable for implantation onto or within the eye. As is commonly known, the circuit depicted in Figure 2 can be implemented on a very small substrate. Since the power levels needed for the sensing, optional filtering, and amplifying functions is very low, the circuit components can be placed very close together. In one implementation of system 100, the substrate needed to perform these functions is on the order of one square millimeter - small enough to the implanted in the eye and coupled to an electronic accommodative IOL.

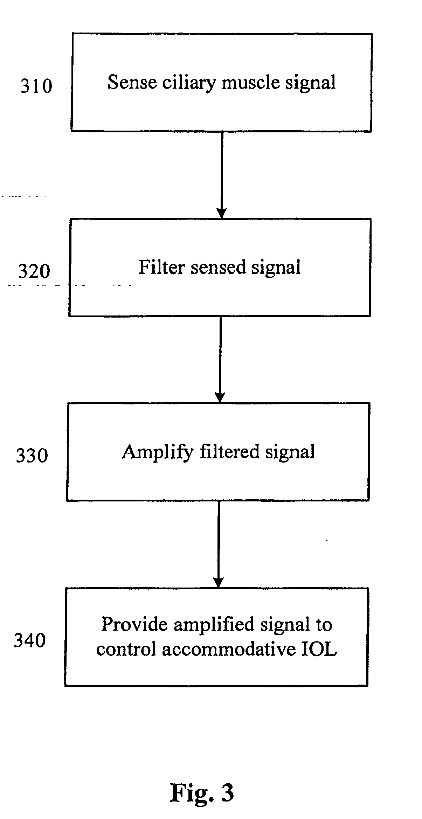

Figure 3 is a flow chart of a method of sensing and amplifying ciliary muscle signals for controlling an accommodative IOL according to the principles of the present invention. In 310, the signal that controls the ciliary muscles is sensed. In 320, the sensed signal is filtered. In 330, the filtered signal is amplified. In 340, the amplified signal is provided to control an accommodative IOL. As previously noted, filtering is optional, and if filtering is used, it can be performed after amplifying.

From the above, it may be appreciated that the present invention provides a circuit for sensing and amplifying the electrical signals that control the ciliary muscles. The present invention thereby provides a control system that can be used to control an accommodative IOL. The present invention is illustrated herein by example, and various modifications may be made by a person of ordinary skill in the art.

Other embodiments of the invention will be apparent to those skilled in the art from consideration of the specification and practice of the invention disclosed herein

## Claims

1. An intraocular lens system comprising:
a control system (100) for an accommodative intraocular lens, the control system comprising:
a sensing circuit (110) for sensing a signal that controls a ciliary muscle of an eye;
an amplifier circuit (130) for amplifying the sensed signal, an output of the amplifier circuit used to control an accommodative intraocular lens; and
at least one electrode (220) electrically coupled to the sensing circuit, wherein the at least one electrode is configured for placement in the vicinity of cranial nerve III, the ciliary ganglion, or the ciliary muscles.

2. The system of claim 1 further comprising:
a filter (120) for filtering a signal.

3. The intraocular lens system of claim 1 wherein the at least one electrode (220) is electrically coupled to an implantable variable resistor that converts movement of a ciliary muscle into an electrical signal.

4. The intraocular lens system of claim 1 further comprising:
an accommodative intraocular lens electrically coupled to the amplifier circuit such that an output of the amplifier circuit (130) controls the accommodative intraocular lens.

5. The intraocular lens system of claim 1 wherein the sensing circuit and the amplifier circuit are located on a single substrate suitable for implantation in the eye.

## Patentansprüche

1. Intraokulares Linsensystem, aufweisend:
ein Steuersystem (100) für eine akkommodative intraokulare Linse, welches Steuersystem aufweist:
einen Sensorkreis (110) zum Lesen eines Signals, das einen Ziliarmuskel eines Auges steuert;
einen Verstärkerkreis (130) zum Verstärken des gelesenen Signals, wobei ein Ausgang des Verstärkerkreises zur Steuerung einer akkomodativen intraokularen Linse zur Verwendung steht; und
mindestens eine Elektrode (220), die elektrisch an den Sensorkreis geschaltet ist, wobei die mindestens eine Elektrode dazu konfiguriert ist, in der Nähe eines Hirnnervs III, des Ziliarganglions oder von Ziliarmuskeln platziert zu werden.

2. System nach Anspruch 1, ferner aufweisend:
einen Filter (120) zum Filtern eines Signals.

3. Intraokulares Linsensystem nach Anspruch 1, wobei die mindestens eine Elektrode (220) elektrisch an einem implantierbaren, variablen Widerstand gekoppelt ist, um eine Bewegung eines Ziliarmuskels in ein elektrisches Signal zu konvertieren.

4. Intraokulares Linsensystem nach Anspruch 1, ferner aufweisend:
eine an dem Verstärkerkreis elektrisch angeschlossene akkommodative intraokulare Linse, derart, dass ein Ausgang des Verstärkerkreises (130) die akkomodative intraokulare Linse steuert.

5. Intraokulares Linsensystem nach Anspruch 1, wobei der Sensorkreis und der Verstärkerkreis auf einem einzelnen Substrat platziert sind, das für eine Implantation in das Auge geeignet ist.

## Revendications

1. Système de lentille intraoculaire comprenant :
un système de commande (100) pour une lentille intraoculaire d'accommodation, le système de commande comprenant :
un circuit de détection (110) pour détecter un signal qui commande un muscle ciliaire d'un oeil ;
un circuit d'amplification (130) pour amplifier le signal détecté, une sortie du circuit d'amplification étant utilisée pour commander une lentille intraoculaire d'accommodation ; et
au moins une électrode (220) couplée électriquement au circuit de détection, dans lequel la au moins une électrode est configurée pour un placement dans le voisinage du nerf crânien III, du ganglion ciliaire ou des muscles ciliaires.

2. Système selon la revendication 1, comprenant en outre :
un filtre (120) pour filtrer un signal.

3. Système de lentille intraoculaire selon la revendication 1, dans lequel la au moins une électrode (220) est couplée électriquement à une résistance variable implantable qui convertit le mouvement d'un muscle ciliaire en un signal électrique.

4. Système de lentille intraoculaire selon la revendication 1, comprenant en outre :
une lentille intraoculaire d'accommodation couplée électriquement au circuit d'amplification de telle sorte qu'une sortie du circuit d'amplification (130) commande la lentille intraoculaire d'accommodation.

5. Système de lentille intraoculaire selon la revendication 1, dans lequel le circuit de détection et le circuit d'amplification sont situés sur un substrat unique approprié pour une implantation dans l'oeil.
